(19) Europäisches Patentamt
European Patent Office
Office européen des brevets

(11) Publication number: **0 185 979**
**A1**

(12) # EUROPEAN PATENT APPLICATION

(21) Application number: 85115328.8

(22) Date of filing: 03.12.85

(51) Int. Cl.[4]: C 07 D 401/04
C 12 P 17/10, C 12 P 17/16
C 12 P 1/06
//C12R1/465, C12R1/47

(30) Priority: 11.12.84 JP 262317/84

(43) Date of publication of application: 02.07.86 Bulletin 86/27

(84) Designated Contracting States: AT BE CH DE FR GB IT LI LU NL SE

(71) Applicant: MICROBIAL CHEMISTRY RESEARCH FOUNDATION
14-23, Kamiosaki 3 Chome Shinagawa-ku
Tokyo 141(JP)

(72) Inventor: Umezawa, Hamao, Prof.
23, Toyotama-kita 4-chome
Nerima-ku Tokyo(JP)

(72) Inventor: Takeuchi, Tomio
1-11, Higashigotanda 5-chome
Shinagawa-ku Tokyo(JP)

(72) Inventor: Hamada, Masa
26, Naito-cho 1-chome
Shinjuku-ku Tokyo(JP)

(72) Inventor: Sawa, Tsutomu
6-7, Ryosei 4-chome
Ayase-city Kanagawa Prefecture(JP)

(72) Inventor: Naganawa, Hiroshi
17, Denenchofu-honcho 3-chome
Ohta-ku Tokyo(JP)

(72) Inventor: Isshiki, Kunio
2-13-1332, 3910, Oba
Fujisawa-city Kanagawa Prefecture(JP)

(74) Representative: Becker, Heinrich Karl Engelbert, Dr. et al,
HOECHST AKTIENGESELLSCHAFT Central Patent Department P.O. Box 80 03 20
D-6230 Frankfurt am Main 80(DE)

(54) **A novel substance having anti-tumor activity, a microbiological process for the preparation thereof and its use as a medicament.**

(57) The present invention relates to a novel compound analogous to the antibiotic streptonigrin having anti-tumor activity and to a microbiological process for producing such compound. The compound exhibits relatively low cytotoxicity and can, therefore, by used as an effective and safe anti-tumor agent.

EP 0 185 979 A1

The present invention relates to a novel compound analogous to streptonigrin having anti-tumor activity, and a process for producing such compound.

The compound known to be analogous to the compound of the present invention is the antibiotic streptonigrin that is obtained from the culture solution of Streptomyces flocculus and which has the formula:

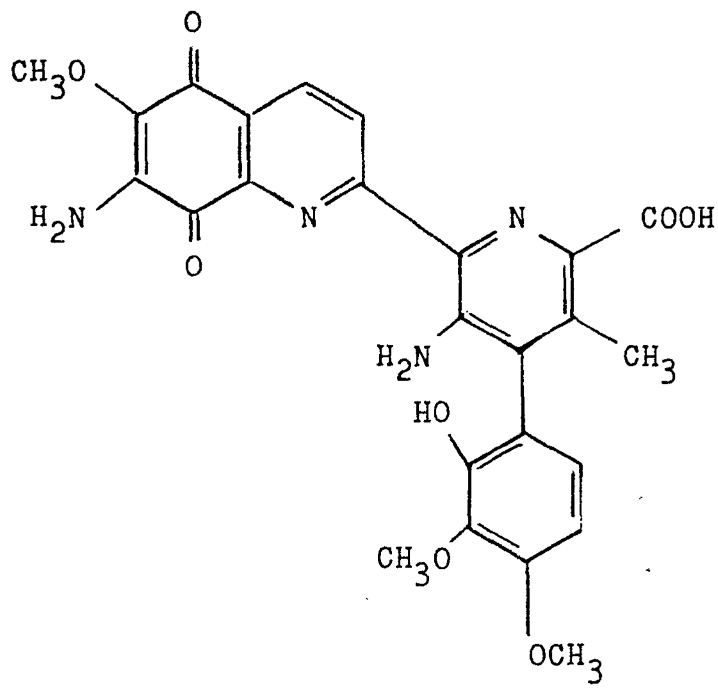

(see, for example, ANTIBIOTICS ANNUAL, 950 - 953, 1959 - 1960).

This antibiotic exhibits strong anti-tumor activity and intensive clinical experiments have been conducted in the United States of America and European countries.

The clinical experiments with streptonigrin have centered around the use of this anti-tumor agent in combination with vincristine, prednisone or bleomycin so as to cure lymphosarcoma, reticulum cell sarcoma, malignant melanoma and non-Hodgkin's lymphoma (in infants). However, because of its high toxicity, streptonigrin has yet to be commercialized as a therapeutic.

With a view to reducing the toxicity of streptomycin while retaining its strong anti-tumor activity, a variety

of streptonigrin derivatives have been synthesized but no satisfactory compounds have been offered.

In order to locate compounds of low toxicity that retain the high anti-tumor activity of streptonigrin, the cultures of various microorganisms have been screened. As a result, it has been found that a microorganism of the genus Streptomyces produces a compound of formula (I)

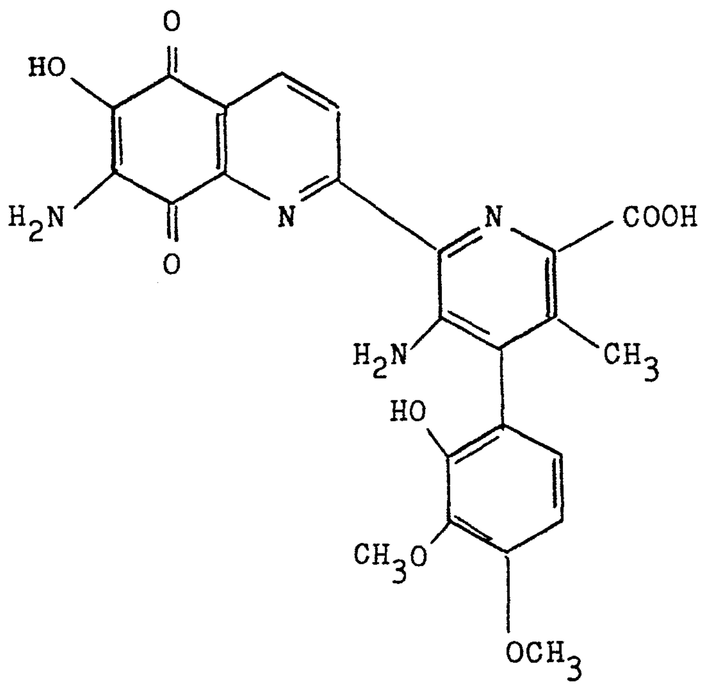

and that this compound has a lower cytotoxicity than streptonigrin.

The compound in accordance with the present invention is very close to streptonigrin. It differs from streptonigrin in that the methoxy group at 6-position of the quinoline nucleus is converted to a hydroxyl group. This novel compound was denominated as substance MG883-12F2.

Substance MG883-12F2 is slightly inferior to streptonigrin in the anti-tumor activity but, as mentioned above, this substance has a lower cytotoxicity and may potentially be used as an anti-tumor agent. Substance MG883-12F2 is also a useful intermediate for synthesizing a variety of desired derivatives by making use of the 6-

positioned hydroxyl group on the quinoline nucleus.

The anti-tumor activity of substance MG883-12F2 can be confirmed by measuring its ability to inhibit the growth of known cultured cells. For example, the median inhibition dose, $ID_{50}$, of substance MG883-12F2 against cultured mouse leukemia cell P388 was 0.5 mg/ml.

In accordance with the second aspect of the invention, there is provided a microbiological process for producing the substance MG883-12F2.

This process comprises culturing on a nutrient medium a microorganism of the genus Streptomyces or one of its mutants having the ability to produce the substance MG883-12F2 and recovering the desired substance from the culture.

The microorganism that can be used in the present invention may be any species of the genus Streptomyces that has the ability to produce the substance MG883-12F2. A specific example is the Actinomycetes that was isolated from the soil and which was deposited with the Fermentation Research Institute, Agency of Industrial Science and Technology on February 20, 1984 under FERM-P-No. 7461 with the designation "Substance MG883-12F2".

The microorganism may be cultured on any media that contain nutrient sources that can be utilized by microorganisms capable of producing substance MG883-12F2. Suitable nutrient media are those which are commonly used in the cultivation of Actinomyces. Usable carbon sources include glycerin, glucose, sucrose, maltose, dextrin, starch and fats and oils. Usable nitrogen sources include organics such as soybean meal, cottonseed oil cake, meat extract, peptone, dried yeast, yeast extract and corn steep liquor, and inorganics such as ammonium salts and nitrates, for example, ammonium sulfate, sodium nitrate and ammonium chloride. If necessary, inorganic salts such as sodium chloride, potassium chloride, phosphates and heavy metal salts may be added to the medium. In order to prevent foaming during fermentation, suitable

anti-foaming agents such as silicone and soybean oil may be added in suitable amounts as effected in the usual practice.

Aerobic liquid submerged culture is the most appropriate method as is commonly employed in the production of antibiotics. The suitable temperature for incubation ranges from 20 to 35°C, with the 25 - 30°C range being preferred. The amount of substance MG883-12F2 produced by aeration and agitation, reaches a peak in 3 - 4 days.

The above procedures yield a culture in which substance MG883-12F2 has accumulated. The most part of this substance is present in the supernatant of the culture.

Any suitable method may be used to recover the substance MG883-12F2 from the culture. One method depends on the principles of extraction, and more specifically, substance MG883-12F2 in the culture supernatant may be extracted with water-miscible solvents for MG883-12F2, such as ethyl acetate, butyl acetate and chloroform.

Another method that can be used to recover substance MG883-12F2 from the culture depends on adsorptive processes, wherein an already liquid material containing substance MG883-12F2, such as a culture filtrate or an extract that has been obtained by the first method is subjected to column chromatography or liquid chromatography using a suitable adsorbent, say, silica gel or (R)Dia-Ion HP20 (Mitsubishi Chemical Industries Limited), and the adsorbed substance MG883-12F2 is eluted with a suitable solvent. The eluate is subsequently concentrated to dryness under vacuum to obtain a crude product of the end substance as a red powder.

The crude produce of substance MG883-12F2 may be purified by performing the required number of cycles of the extraction and adsorption processes described above, which may be combined as required, optionally followed by recrystallization. Suitable purification techniques that may be used in combination include column chromatographic processes using adsorbents and gel permeation media such

as silica gel, (R)Sephadex LH 20 and (R)Dia-Ion HP 20 (Mitsubishi Chemical Industries Limited), liquid chromatography using suitable solvents, the counter-current distribution method and thin-layer chromatography. A specific purification process may proceed as follows: the crude powder of substance MG883-12F2 is dissolved in a small amount of chloroform; the solution is loaded onto a silica gel column and fractions are eluted with a suitable solvent; those fractions are containing the desired substance are combined and concentrated under vacuum; the concentrate is subjected to thin-layer chromatography and the desired components are recovered to obtain a substantially homogeneous product. This product may be further purified by high-performance liquid chromatography or crystallization from a suitable solvent.

The MG883-12F2 producing microorganism of the genus Streptomyces that was used in the Example shown later in this specification has the following mycological properties. This specific microorganism is an Actinomycetes isolated by the Institute of Microbial Chemistry in April 1983 from the soil sampled in Setagaya-ku, Tokyo, Japan, and is assigned the strain number MG883-12F2.

1. Morphology

MG883-12F2 as observed under a microscope has spiral aerial mycelia extending from branched aerial hyphae, but no whirl is observed. Mature spore chains have a detectable chain of not less than 10 spores. The spore is in the size range of about 0.4 - 0.5 x 0.8 - 0.9 μm. The spores are smooth-surfaced.

2. State of growth on media

In the following description, color standards are bracketed and they are in accordance with Color Harmony Manual of Container Corporation of America.

(1) Sucrose-nitrate agar medium (incubated at 27°C)

Adhering white to brown-white ⌊5ba, Shell Pink⌋ aerial mycelia form on a colorless to pale yellow growth. No soluble dye observed.

(2) Glucose-asparagine agar medium (incubated at 27°C)

Adhering white to yellowish gray ⌊3ba, Pearl⌋ aerial mycelia form on a pale yellow ⌊2gc, Bamboo - 21c Honey Gold⌋ growth. No soluble dye observed.

(3) Glycerin-asparagine agar medium (ISP-Medium 5, incubated at 27°C)

Adhering white to brown-white aerial mycelia form on a pale yellow brown ⌊31c, Lt Amber - 31e Cinnamon⌋ growth. No soluble dye observed.

(4) Starch-inorganic salt agar medium (ISP-Medium 4, incubated at 27°C)

Adhering brown-white ⌊3ba, Pearl - 5ba, Shell Pink⌋ aerial mycelia form on a pale yellowish brown ⌊21e, Mustard⌋ growth. No soluble dye observed.

(5) Tyrosine agar medium (ISP-Medium 7, incubated at 27°C)

Adhering white to brown-white aerial mycelia form on a pale yellowish brown ⌊21e, Mustard⌋ to pale brown ⌊31g, Adobe Brown⌋ growth. No soluble dye observed.

(6) Nutrient agar medium (incubated at 27°C)

Adhering white aerial mycelia form on a pale yellow-orange ⌊2gc, Bamboo⌋ to pale yellowish brown ⌊2ng, Dull Gold⌋ growth. No soluble dye observed.

(7) Yeast-malt agar medium (ISP-Medium 2, incubated at 27°C)

Adhering yellowish gray ⌊2ca, Lt Ivory⌋ aerial mycelia form on a pale yellow-brown ⌊2ne, Mustard Gold⌋ to dull yellow-orange ⌊3pg, Golden Brown⌋ growth. No soluble dye observed.

(8) Oatmeal agar medium (ISP-Medium 3, incubated at 27°C)

Adhering brown-white aerial mycelia form on a colorless to pale yellow growth. No soluble dye observed.

(9) Glycerine-nitrate agar medium (incubated at 27°C)

Adhering white to yellowish gray ⌊3ba, Pearl⌋ aerial mycelia form on a pale yellow to pale brown ⌊31c, Lt Amber⌋ growth. No soluble dye observed.

(10) Starch agar medium (incubated at 27°C)

White adhering aerial mycelia form on a colorless growth. No soluble dye observed.

(11) Calcium malate agar medium (incubated at 27°C)

Adhering white to brown-white aerial mycelia form on a colorless to pale yellowish brown [2gc, Bamboo] growth. No soluble dye observed.

(12) Cellulose (incubated at 27°C)

Adhering brown-white aerial mycelia form on a colorless growth. No soluble dye observed.

(13) Gelatin stab culture

In incubation on a simple gelatin medium at 20°C, white adhering aerial mycelia form on a colorless to pale yellowish brown [2ne, Mustard Gold] growth, with no soluble dye observed. In incubation on a glucose-peptone gelatin medium at 27°C, white adhering aerial mycelia form on a pale yellow [1-1/2 ea, Lt Yellow - 2ea, Lt Wheat] to pale brown [31c, Lt Amber - 31e, Cinnamon] growth. Soluble dye with a slight brown tinge observed.

(14) Skimmed cow's mild (incubated at 37°C)

Adhering brown-white aerial mycelia form on a pale pink [4gc, Nude Tan - 5gc, Peach Tan - 5ie Copper Tan] growth. Soluble dye with a brown tinge observed.

3. Physiological properties

(1) Growth temperature range

Growth test was conducted on glucose-asparagine agar (1.0% glucose, 0.05% L-asparagine, 0.05% potassium hydrogen-phosphate, 3.0% agar, pH 7.0) at varying temperatures, 20°C, 24°C, 27°C, 30°C,, 37°C and 50°C. Except at 50°C, the microorganism grew at each of the temperatures tested, but an optimal temperature would be in the range of 30 - 37°C.

(2) Liquifaction of gelatin (on 15% simple gelatin at 20°C, or glucose-peptone gelatin at 27°C)

Growth on a simple gelatin medium was observed for 20 days but no liquefaction occured at all. With a glucose-peptone gelatin-medium, liquefaction started at

about 6 days of incubation, and the degree of liquefaction was moderate to weak.

(3) Hydrolysis of starch (incubated either on starch-inorganic salt agar medium or on starch agar medium at 27°C)

Hydrolysis of starch was observed in neither medium.

(4) Coagulation and peptonization of skimmed cow's milk (incubated on skimmed cow's milk at 37°C)

Coagulation of skimmed cow's milk started at about 10 days of incubation and was soon completed, followed by peptonization. The degree of coagulation and peptonization was moderate to strong.

(5) Formation of melanine-like pigment (with tryptone-yeast broth, ISP-Medium 1, peptone-yeast-iron agar, ISP-Medium 6, or tyrosine agar, ISP-Medium 7 at 27°C)

No melanine-like pigment observed in either medium.

(6) Utilization of carbon sources (on Pridham-Gottlieb agar medium, ISP-Medium 9, at 27°C)

The microorganism grew utilizing D-xylose, glucose and D-mannitol, but not utilizing sucrose, inositol, rhamnose or raffinose. The microorganism would probably not utilize D-fructose, but no definite conclusion can be reached about the utilization of L-arabinose.

(7) Dissolution of calcium malate (on calcium malate agar at 27°C)

No dissolution of calcium malate was observed.

(8) Reduction of nitrate (in aqueous solution of peptone containing 0.1% $KNO_3$, ISP-Medium 8, at 27°C)

Both positive and negative cases occurred in cyclic test.

The above observations can be summarized as follows: morphologically, strain MG883-12F2 has no sporangium or whirl and has spiral aerial mycelia. The microorganism has smooth-surfaced spores. It grows on a variety of media, providing white to brown-white or yellowish gray aerial mycelia on pale yellow to pale yellowish brown or pale brown growths.

Soluble dyes are seldom observed, and if they occur they are tinged with brown. The formation of a melanine-like pigment is negative in all of the media used. No hydrolysis of starch is observed. The ability of the microorganism to decompose protein is such that it weakly liquefieds gelatin and coagulates and peptonizes skimmed cow's milk moderately to strongly.

The cells of this microorganism contain an LL-form of 2,6-diaminopimelic acid. This fact, taken together with the above described properties, clearly shows that strain MG883-12F2 belongs to the genus Streptomyces.

Utilizing these mycological properties as a guide, the following three known streptomycetes are considered to be homologous to MG883-12F2: Streptomyces albus in International Journal of Systematic Bacteriology, 19, 401, 1969; Streptomyces almquisti in International Journal of Systematic Bacteriology, 19, 403, 1969 and Streptomyces rangoon in International Journal of Systematic Bacteriology, 19, 472, 1969, the last two microorganisms were both found to be identical with Streptomyces albus.

In order to confirm this assumption, the present inventors obtained the ISP strains of these three homologues and compared them with MG883-12F2. The results of comparison are shown in the following table.

| | MG883-12F2 | Streptomyces albus IMC S-0296 (ISP5313) | Streptomyces almquisti IMC S-033 (ISP5447) | Streptomyces rangoon IMC S-0325 (ISP5452) |
|---|---|---|---|---|
| Whirl formation | − | − | − | − |
| Spiral formation | + | + | + | + |
| Spore surface | smooth | smooth | smooth | smooth |
| Color of aerial mycelium | white-brown white, yellowish gray | white − yellowish white | white − yellowish white | white − yellowish gray |
| Color of growth | pale yellow − pale yellow brown, yellow brown | pale yellow − pale yellow brown | pale yellow − pale yellow brown | pale yellow − pale yellow brown, yellow brown |
| Soluble dye | − | − | − | − |
| Formation of melanine-like pigment | | | | |
| ISP-medium 1 | − | − | − | − |
| ISP-medium 6 | − | − | − | − |
| ISP-medium 7 | − | − | − | − |
| Hydrolysis of starch | − | − | − | − |
| Coagulation of cow's milk | + | + | + | + |
| Peptonization of cow's milk | + | + | + | + |
| Liquefaction of gelatin | | | | |
| Simple gelatin | − | − | − | ±(weak) |
| Glucose-peptone gelatin | + | + | + | + |
| Reduction of nitrate | (positive or negative in another test) | − | − | ± (positive or negative in another test) |
| Utilization of carbon sources | | | | |
| glucose | + | + | + | + |
| L-arabinose | d | d | d | d |
| D-xylose | + | ± | + | ± |
| D-fructose | ∓ | ± | ± | ∓ |
| sucrose | − | − | − | − |
| innositol | − | − | d | − |
| rhamnose | − | − | − | − |
| raffinose | − | − | − | − |
| D-mannitol | + | + | + | + |

+: utilized ±: probably utilized d: unclear ∓: probably not utilized −: not utilized

As the table shows, MG883-12F2 is very similar in mycological properties to each of the three strains of Streptomyces albus, i.e. Streptomyces albus, Streptomyces almquisti and Streptomyces rangoon. Streptomyces albus var. bruneomycini was reported as a microorganism capable of producing streptonigrin (alias bruneomycin) produced by MG883-12F2 [see Antibioki (Moscow), 1969, 14(11), 997-1000 (Rus.)].

With all the observations taken together, MG883-12F2 is believed to be most homogolous to Streptomyces albus, and the inventors identified said strain as Streptomyces albus MG883-12F2.

The following Example is provided for further illustration of the present invention.

Example

(1) Preparation of seed culture

A medium (110 ml) containing 1% glucose, 0.3% beef extract (Kyokuto), 0.5% yeast extract (Difco), 0.5% triptose (Difco) and 0.15% agar powder (Difco) at pH 7.0 was poured into a 500-ml Erlenmeyer flask. The sterilized medium was inoculated with a loopful of a slant culture of strain MG883-12F2. The medium was shake-cultured in a rotary shaker at 27°C for 3 days.

(2) Fermentation

A medium having the same composition as that used above was inoculated with 3% of the seed culture prepared in (1) and shake-cultured at 27°C for 4 days.

(3) Recovery of substance MG883-12F2

The culture obtained in (2) was centrifuged and 5 liters of the supernatant was adjusted to a pH of 3.5 with 1N HCl. The supernatant was subjected to extraction with 6,600 ml of ethyl acetate, dehydrated over anhydrous sodium sulfate and evaporated to dryness in vacuo to obtain 560 mg of a powder. The powder was dissolved in 1.5 ml of methanol, mixed with 1.5 g of silica gel (Kieselgel(R) 60 of Merck, 70 - 230 mesh) and dried in a desiccator in vacuo. The dried mixture was loaded onto a 10-g

silica gel column filled with chloroform. After elution with chloroform/methanol(100/3), the eluate was subjected to column chromatography (200 ml) on Sephadex LH-20 column and eluted with methanol. The fractions were subjected to analytical high-performance liquid chromatography ((R)Nucleosil 5c18 of Macherey-Nagel, 4.5 mm$^{\varnothing}$ x 250 mm) with acetonitrile/(1% citric acid + 5% potassium acetate, pH 6.0) (60/60) used as an eluant. The combined fractions were concentrated and subjected to another run of high-performance liquid chromatography (Nucleosil 5c18, 20 mm$^{\varnothing}$ x 300 mm) with an eluant having the same composition as used above. The eluate was loaded onto another analytical HPLC column (R)Nucleosil 5c18, 4.5 mm$^{\varnothing}$ x 250 mm) and fractions containing substance MG883-12F2 were extracted with ethyl acetate at pH 3.5. The extract was purified by another run of (R) Sephadex LH-20 column chromatography (1.5 mm$^{\varnothing}$ x 20 cm) to obtain a pure form of substance MG883-12F2 that exhibited a single peak upon analysis by a 5-mg analytical column.

Physiochemical properties:

The substance MG883-12F2 had a melting point of 177-181°C (with decomposition) and the following IR and UV spectra.

IR absorption spectrum (KBr tab.): $\nu_{max}^{KBr}$ $cm^{-1}$

3440, 3370, 3230, 2930, 1715, 1675, 1610, 1585, 1500, 1460, 1440, 1380, 1350, 1230, 1190, 1075, 1050, 1025, 995;

NMR: $\delta$ ppm ($CO_3OD$) 2.35 (3H, S), 3.83 (3H, S), 3.97 (3H, S), 5.98 (2H, bs), 6.52 ((1H, d, J=8.5 Hz), 6.65 (1H, , J=8.4 Hz), 7.56 (1H, bs), 7.85 (1H, bs), 8.41 (1H, d, J=9 Hz), 8.95 (1H, d, J=9 Hz), 10.9 (1H, br);

$^{13}C$ NMR: $\delta$ ppm ($CDCl_3$) 17.3, 60.2, 60.6, 109.4, 114.4, 125.9, 126.2, 127.7, 130.4, 133.8, 134.2, 134.7, 137.1, 137.5, 139.2, 141.0, 145.3, 147.7, 148.9, 151.4, 166.9, 185.5, 177.2, 181.0.

Claims:

1. The compound represented by the formula (I):

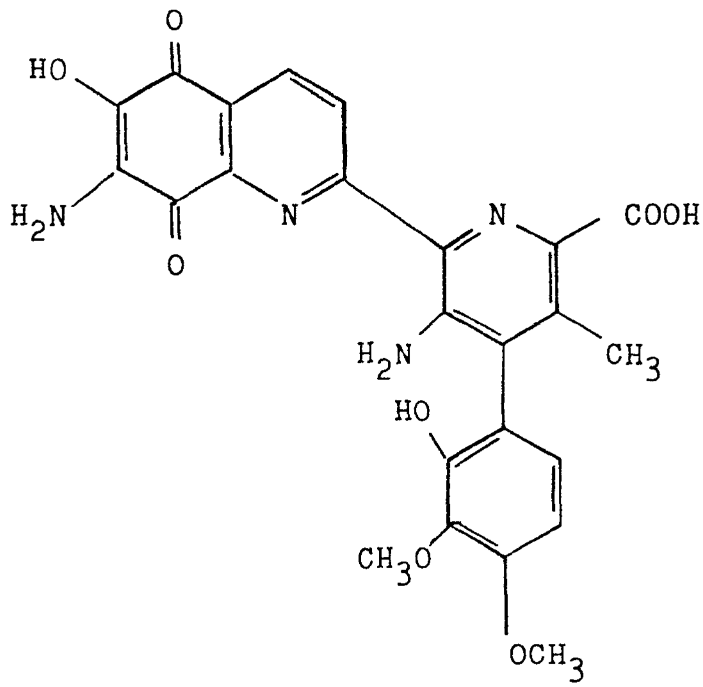

2. A process for producing the compound according to claim 1 which comprises first culturing in a nutrient medium a microorganism of the genus Streptomyces or one of its mutants that has the ability to produce the compound and then recovering the compound from the culture by conventional methods.

3. A process according to claim 2 wherein the microorganism of the genus Streptomyces is Streptomyces albus (FERM-P No. 7461) or one of its mutants.

4. The process as defined in claims 2 or 3 wherein the cultivation is carried through in a liquid submerged culture medium comprising nutritional carbon sources, nitrogen sources and optionally inorganic salts and antifoaming agents at a temperature of from 20 to 35°C and a cultivation time of from 3 to 4 days, and the produced compound is recovered from the culture broth by conventional extraction or adsorptive methods and then purified by chromatography.

5. The process as defined in claim 4 wherein the culture filtrate or extract thereof is subjected to column chromatography.

6. A pharmaceutical composition which comprises the compound as defined in claim 1 as the active ingredient and a pharmaceutically acceptable carrier.

7. Use of the compound as defined in claim 1 for the preparation of a medicament having anti-tumor activity.

8. The strain Streptomyces albus FERM-P No. 7461.

Claims for the contracting state Austria:

1. A process for producing a compound of the formula (I):

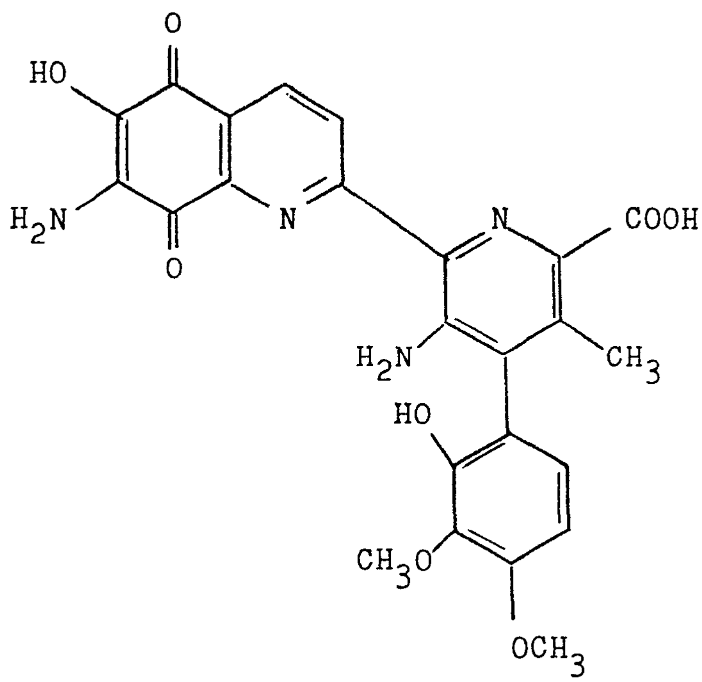

which comprises first culturing in a nutrient medium a microorganism of the genus Streptomyces or one of its mutants that has the ability to produce the compound and then recovering the compound from the culture by conventional methods.

2. A process according to claim 2 wherein the microorganism of the genus Streptomyces is Streptomyces albus (FERM-P No. 7461) or one of its mutants.

3. The process as defined in claims 2 or 3 wherein the cultivation is carried through in a liquid submerged culture medium comprising nutritional carbon sources, nitrogen sources and optionally inorganic salts and antifoaming agents at a temperature of from 20 to 35°C and a cultivation time of from 3 to 4 days, and the produced compound is recovered from the culture broth by conventional extraction or adsorptive methods and then purified by chromatography.

4. The process as defined in claim 4 wherein the culture filtrate or extract thereof is subjected to column chromatography.

European Patent Office

# EUROPEAN SEARCH REPORT

0185979 Application number

EP 85115328.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 64, no. 5, February 28, 1966, Columbus, Ohio, USA<br>TETSUJI KAMETANI, KUNIO OGASAWARA "Synthesis of heterocyclic compounds Streptonigrin and related compounds. 1. Synthesis of 5,6,8-trimethoxy-7--dimethylaminoquinoline and 7-amino--6-hydroxy-5,8-quinolinedione" column 6611, abstract-no. 6611e<br>& YAKUGAKU ZASSHI 85(1), 985-90 (1965)<br>-- | 1 | C 07 D 401/04<br>C 12 P 17/10<br>C 12 P 17/16<br>C 12 P 1/06//<br>C 12 R 1/465<br>C 12 R 1/47 |
| A | CHEMICAL ABSTRACTS, vol. 87, no. 23, December 5, 1977, Columbus, Ohio, USA<br>RAO, KOPPAKA V. "Streptonigrin and related compounds III. Synthesis and microbiological activity of detrioxyphenylstreptonigrin and analogs" page 596, column 1, abstract-no. 184 343q<br>& J. Heterocycl. Chem. 1977, 14(4), 653-9<br>-- | 1 | TECHNICAL FIELDS SEARCHED (Int. Cl.4)<br>C 07 D 401/00<br>C 12 P |

The present search report has been drawn up for all claims

| Place of search | Date of completion of the search | Examiner |
|---|---|---|
| VIENNA | 06-03-1986 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent Office

# EUROPEAN SEARCH REPORT

0185979
Application number

EP 85115328.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 92, no. 7, February 18, 1980, Columbus, Ohio, USA<br>RAO, KOPPAKA V.; KUO, HSIEN-SAW "Streptonigrin and related compounds. IV. Precursors for the A ring" page 655, column 1, abstract-no. 58 578k<br>& J. Heterocycl. Chem. 1979, 16(6), 1241-8<br>-- | 1 | |
| A | CHEMICAL ABSTRACTS, vol. 100, no. 13, March 26, 1984, Columbus, Ohio, USA<br>GERWICK, WILLIAM J.; GOULD, STEVEN J.; FONOUNI, HOSSEIN "Biosynthesis of streptonigrin. 6. The biosynthesis of streptonigrin from [1-13C]-D-erythrose" page 333, column 2, abstract-no. 99 614w<br>& Tetrahedron Lett. 1983, 24(49), 5445-8<br>-- | 1 | TECHNICAL FIELDS SEARCHED (Int. Cl.4) |

The present search report has been drawn up for all claims

| Place of search | Date of completion of the search | Examiner |
|---|---|---|
| VIENNA | 06-03-1986 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

0185979
Application number

EP 85115328.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 94, no. 25, June 22, 1981, Columbus, Ohio, USA<br>KENDE, ANDREW S.; LORAH, DENNIS P.; BOATMAN, RODNEY J. "A new and efficient total synthesis of streptonigrin" page 582, column 2, abstract-no. 208 676y<br>& J. Am. Chem. Soc. 1981, 103(5), 1271-3<br>-- | 1 | |
| A | CHEMICAL ABSTRACTS, vol. 93, no. 9, September 1, 1980, Columbus, Ohio, USA<br>BASHA, FATIMA Z.; HIBINO, SATOSHI; KIM, DEUKJOON; PYE, WALTER E.; WU, TAI-TEH; WEINREB, STEVEN M. "Total synthesis of streptonigrin" page 629, column 2, abstract-no. 95 110t<br>& J. Am. Chem. Soc. 1980, 102(11), 3962-4<br>---- | 1 | TECHNICAL FIELDS SEARCHED (Int. Cl.4) |

The present search report has been drawn up for all claims

| Place of search | Date of completion of the search | Examiner |
|---|---|---|
| VIENNA | 06-03-1986 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82